# EUROPEAN PATENT SPECIFICATION

(11) **EP 3 325 152 B1**
(45) Date of publication and mention of the grant of the patent: **07.12.2022**
(21) Application number: 16744343.1
(22) Date of filing: 19.07.2016
(51) Int. Cl.: B01L 7/00, C12Q 1/6806, C12Q 1/6869, B01L 3/00

(54) **AUTOMATED SAMPLE TO NGS LIBRARY PREPARATION**
AUTOMATISCHE VORBEREITUNG VON PROBEN ZU NGS-BIBLIOTHEK
PRÉPARATION AUTOMATISÉE DE BIBLIOTHÈQUES DE SÉQUENÇAGE À HAUT DÉBIT À PARTIR D'ÉCHANTILLONS

(30) Priority: 23.07.2015 EP 15178158
(43) Date of publication of application: 30.05.2018
(73) Proprietor: Biocartis NV, 2800 Mechelen (BE)
(72) Inventor: VERGAUWE, Nicolas, 2800 Mechelen (BE); MEERSSEMAN, Geert, 2800 Mechelen (BE)
(74) Representative: Arnold & Siedsma
(86) International application number: PCT/EP2016/067149
(87) International publication number: WO 2017/013103

(56) References cited:
- WO-A2-2012/012779
- Anna-Iris Schiefer: "Evaluation of a novel fully automated PCR-based technique for detection of BRAF-status in FFPE-tissues", , 1 January 2014 (2014-01-01), XP055229934, Retrieved from the Internet: URL:https://media.biocartis.com/biocartis/ documents/141103_AMP-Poster_BRAF-Study_AT_ Wien_AKH_Pathologie_Birner_FINALFINAL.pdf [retrieved on 2015-11-20]
- "Beckman Coulter SPRIworks Systems", , 1 January 2011 (2011-01-01), XP055229930, Retrieved from the Internet: URL:https://www.beckmancoulter.com/wsrport al/bibliography?docname=BR-15981.pdf [retrieved on 2015-11-20]
- BUEHLER B ET AL: "Rapid quantification of DNA libraries for next-generation sequencing", METHODS, ACADEMIC PRESS, US, vol. 50, no. 4, 1 April 2010 (2010-04-01), pages S15-S18, XP026966692, ISSN: 1046-2023 [retrieved on 2010-03-07]
- ALISON S. DEVONSHIRE ET AL: "Application of next generation qPCR and sequencing platforms to mRNA biomarker analysis", METHODS, vol. 59, no. 1, 1 January 2013 (2013-01-01), pages 89-100, XP055228123, US ISSN: 1046-2023, DOI: 10.1016/j.ymeth.2012.07.021
- "The SPRIworks Fragment Library System Simplifying Next Generation Sequencing Workflows SPRIworks Fragment Library System II Fully Automated Fragment Library System for the Roche GS FLX* DNA Sequencer", , 1 January 2010 (2010-01-01), XP055229937, Retrieved from the Internet: URL:https://www.beckmancoulter.com/wsrport al/bibliography?docname=DS-14103.pdf [retrieved on 2015-11-20]

## Description

### TECHNICAL FIELD

The present invention generally relates to cartridges for fully automated processing of biological samples to next-generation sequencing-ready nucleic acid libraries. In particular, the present invention concerns a cartridge comprising a compartment for receiving a biological sample, means for liberating and/or purifying nucleic acids from the received sample and for transporting said nucleic acids to a compartment wherein NGS library can be prepared from said nucleic acids using provided therein reagents. In a particular aspect, the present invention also concerns cartridges for automated preparing a NGS nucleic acid library from a biological sample simultaneously or sequentially with a qPCR assay performed on the same biological sample.

### BACKGROUND OF THE INVENTION

The ever-decreasing costs of high-throughput sequencing, also known as second generation- or next generation-sequencing (NGS) in contrast to much slower classical Sanger sequencing, are gradually bringing this powerful high-coverage diagnostic technique into clinical practice. Currently, there exist many different NGS strategies and platforms, including e.g. the commercially known Illumina (Solexa) sequencing, Roche 454 sequencing, Ion torrent (Proton / PGM) sequencing, or SOLiD sequencing. All of them differ in details but in general involve generation of many short overlapping nucleic acid fragments or "reads", often modified to include sequencing platform-specific adapter sequences. A collection of such fragments prepared from a specific source of a nucleic acid is called a NGS nucleic acid library. A process of preparing such nucleic acid library from a clinical sample is time-consuming and requires multiple preparatory steps. Namely, not only nucleic acids retrieved from a sample have to be sufficiently pure and recovered in a sufficient amount but also, in order to provide satisfactory genetic information coverage, they have to meet often stringent quality criteria and be free of contaminations that could impede either the library preparation step or the sequencing reaction. As a result, NGS library preparation is not only still labor-intensive on its own, but it also requires additional steps of assessing the quantity and quality of either the purified nucleic acid or constructed NGS library prior to sequencing step in order to ensure high-quality results and their correct interpretation. Thus, even despite the tremendous improvements made to make NGS even faster, cheaper, and requiring less manpower, the technique is still relatively expensive and much slower as compared to diagnostic screening via e.g. target specific quantitative or real-time PCR (qPCR).

To date, processing of a clinical sample taken from a patient to a NGS-ready is largely done manually and at least includes the steps of:
1. cell lysis or liquefaction;
2. liberation or purification of the nucleic acids;
3. determination of the quality and quantity of the nucleic acids by any known means in the art, for example absorbance (optical density) measurement, agarose gel electrophoresis, fluorometry with a fluorescent DNA-binding dyes like picogreen, or different types of quality control PCRs (cf. Buehler et al. 2010);
4. preparation of a NGS library according to a strategy depending on the NGS platform of choice; and
5. often but optionally, determination of the quality and quantity of the DNA library by any of the means known in the art.

Some NGS providers like Illumina make their best efforts to simplify the above-explained workflow, example of which is the introduced in January 2015 by Illumina NeoPrep that automates the library preparation step (step 4) from a manually isolated and quality-verified DNA. In addition, the automatic SPRIworks Fragment Library System II uses cartridges for sample liberation and purification, and NGS library generation (cf. Beckman Coulter SPRiWorks systems 2011 and 2010). However, to date there is no protocol for direct, fast, and automated sample-to-NGS-ready library processing, and especially not such that provides for concomitant nucleic acid quality and quantity assessment.

The present invention addresses this and other needs by providing a fluidic cartridge for automated processing of a biological sample of practically any type (including blood, stool, urine, FFPE, swab, etc.) to an NG sequencing ready library. The cartridge of the present invention comprises all the means necessary for automated sample liquefaction, followed by nucleic acid liberation and/or purification, and then preparation of a NGS-ready library from at least a part of the liberated and/or purified nucleic acid without any manual human intervention from the moment the sample is introduced into the cartridge. Importantly, the cartridge of the invention also provides all the means necessary for automated qPCR analysis, which can be performed in parallel with or sequentially with respect to the NGS library preparation from another portion of the same liberated and/or purified nucleic acid of from the NGS library itself. Such qPCR can be a quality control (QC) qPCR for assessing the quality of nucleic acids purified from the sample provided into the cartridge prior to library preparation, or for assessing the quality of library after it has been prepared and before subjecting it to NGS analysis. Alternatively, such qPCR can be an assay qPCR for screening target actionable mutations in a clinical sample. qPCR quantification may allow improvements to current NGS workflows, including reducing the amount of library DNA required, increasing the accuracy in quantifying amplifiable DNA, and avoiding amplification bias by reducing or eliminating the need to amplify DNA before sequencing (Buehler *et al.* 2010). Related to transcriptional (Devonshire *et al.* 2013) or genomic mutation biomarkers (Schiefer *et al.* 2014), high-throughput PCR and sequencing platforms will provide increasing depth to our understanding of the genome and transcriptome in a broad range of complex pathologies.

By automatically and concomitantly generating (a) a NGS-ready library, together with (b) its quality evaluation, and performing (c) target qPCR screening from one and identically-processed biological sample, one would not only save time and trained human resources but also would ensure better management of precious and limited in amount clinical samples. A further advantage of such solution would be the ability to immediately use the information obtained from a real-time-monitored QC qPCR reaction for an indication of nucleic acid quantity and quality, and thus its suitability for NGS library generation. A further advantage would be the ability to directly compare the assay qPCR results and the results obtained from a NGS analysis of the library concomitantly generated with and on the same machine as said qPCR, either at the same time of (i.e. simultaneously) or just shortly after (i.e. sequentially), and, importantly, by using the same pool of identically processed nucleic acid. Furthermore, by thus removing the usual temporal separation between a qPCR analysis and stabilization of the free nucleic acids in a form of a library, one could further minimize any potential analytical dichotomies between qPCR and NGS results, which stem from prolonged storage and potential degradation of biological samples or free nucleic acids.

Thus, the cartridges and methods of the present invention not only allow automated sample-to-NGS-library-generation together with potential verification of the sample's or library's quality, but further provide means for immediate high-depth discovery of driver mutations in advance of NGS-in-depth genomic analysis. This and other advantages of the present invention are presented in continuation.

### SUMMARY OF THE INVENTION

The present invention is defined in the appended independent claims. Preferred embodiments are defined in the dependent claims. In particular, the present invention concerns a cartridge for automated processing of a biological sample, the cartridge comprising
- a sample compartment for receiving a biological sample;
- means for liberating or purifying nucleic acid from the biological sample received in the sample compartment, said means capable of entering in fluid communication with said sample compartment;
- a library compartment for preparing a nucleic acid library positioned downstream of the sample compartment and of the means for liberating or purifying nucleic acid, the library compartment configured to receive at least a portion of the liberated or purified nucleic acid and comprising reagents for preparing a nucleic acid library.

In a preferred embodiment, the above-described cartridge is characterized in that the reagents for preparing a nucleic acid library allow:
- - generation of nucleic acid fragments from the portion of the liberated or purified nucleic acid received into the library compartment, and
- - attaching oligonucleotide adapters to at least one, preferably both ends of said generated nucleic acid fragments.

In a further aspect, the present invention also provides an automated method for preparing a next-generation sequencing (NGS) nucleic acid library from a biological sample, said method comprising the steps of:
a) receiving a biological sample;
b) liberating or purifying at least a part of nucleic acids from the received biological sample;
c) providing at least a portion of the liberated or purified nucleic acid into a library compartment for preparing a NGS nucleic acid library and comprising reagents for preparing a nucleic acid library; and
d) preparing a NGS nucleic acid library;
wherein at least the steps b) to d) are performed on an automated system.

Finally, the invention also provides uses of cartridges according to the invention for automated preparation of a next-generation sequencing (NGS) library from a biological sample.

### DEFINITIONS

The term *"quantitative PCR"* or simply *"qPCR"* is herein given the definition of a laboratory technique based on the polymerase chain reaction (PCR), which is used to amplify and simultaneously detect or quantify a targeted DNA molecule. In contrast to standard PCR where the product of the reaction is detected at its end, i.e. after thermocycling has finished, the key feature of qPCR is that the DNA product is being detected during thermocycling as the reaction progresses in "real time"; hence, the alternative name of qPCR *"real-time PCR".* There currently exist many different types of qPCRs. For example, when starting with a reverse transcription (RT) step, qPCR can be used to quantify numbers of messenger RNAs and is then called a reverse transcriptase qPCR or an RT-qPCR. As used herein the terms *"quantitative PCR"* or simply "*qPCR*" will be employed with preference over the term *"real-time PCR"* or *"RT-PCR"* in order to avoid confusion with *reverse transcription PCR,* also frequently abbreviated as RT-PCR. Most qPCRs use one of the two most common methods for detecting the product amplification in real-time: (a) intercalation of non-specific fluorescent dyes with any double-stranded DNA, or (2) sequence-specific DNA probes consisting of oligonucleotides that are labelled with a fluorescent reporter which permits detection only after hybridization of the probe with its complementary target sequence. The fluorescent signals generated during thermocycling are detected by an appropriate optical detection system and tracked from the moment they pass the background threshold till the reaction reaches plateau. The copy number of the target sequences can be estimated using either relative or absolute quantification strategy, typically by analyzing the shape of the obtained amplification curve (standard curve strategy) or by determining when the signal rises above some threshold value (often called the Ct value, but sometimes also Cp value or Cq value). In relative quantification, the target nucleic acid levels estimated in a given sample using the Ct or standard curve analysis are expressed as relative to values obtained for the same target in another reference sample, for example, an untreated control sample. Conversely, in absolute quantification the qPCR signal is related to input copy number using a standard curve or can also be calculated according to a more recent digital PCR method. For the moment being, the first strategy is still more prevalent and bases the estimation of the target DNA amount by comparing the obtained values with a previously made standard curve. These and other qPCR quantification strategies are broadly known in the art and their calculation can differ in smaller or greater depending on a given application and a qPCR system.

As used herein, the term *"means for performing quantitative PCR"* shall be understood as minimum necessary arrangement of reagents and elements for performing a qPCR. They will usually include any reagents allowing detectable in real time PCR thermocycling of a nucleic acid template received from a source of nucleic acid. Such reagents include but depending on the type of qPCR are not limited to a PCR-grade polymerase, at least one primer set a detectable dye or a probe, dNTPs, PCR buffer etc. Further, the *"means for performing quantitative PCR"* will usually also include any standard known in the art minimal assembly of parts, which usually includes but is not limited to the following: (1) a suitable compartment (further referred to as a *"a thermocycling qPCR compartment*") where the real time-detectable thermocycling can take place. Such compartments can e.g. be formed by a chamber suitable for amplifying nucleic acids, i.e. made from appropriate material and providing for sufficient internal temperature regulation, and also comprising at least one wall allowing real-time detection of signals generated during such amplification, e.g. a wall transparent to light. Further, (2) means for varying temperature in this chamber or other compartment, as broadly known from various existing thermocycling machines. Then, (3) means for detecting the signals generated during the qPCR thermocycling, like an optical detector coupled to a computer etc. In brief, such minimal assembly will normally include any known in the art system or systems capable of initiating and maintaining the thermocycling reaction in the thermocycling qPCR compartment, adjusting and regulating the temperature to ensure stable thermocycling conditions therein etc.; further, it will also include any appropriate detection device or devices, means for data processing (e.g. a computer alternatively connected to a database), and output systems allowing to read and monitor the thermocycling of the qPCR reaction in real-time (usu. a computer screen displaying the reaction progress in an appropriate graphic user interface); as well as any software packages suitable for operating the machinery and/or displaying and possibly also aiding the interpretation of the obtained results.

Further, as used herein, the term *"sequencing library*" or *"sequencing nucleic acid library*" refers to a set of polynucleotides, most often of DNA type, that are ready for sequence analysis, in particular using any of the currently known next-generation sequencing (NGS) strategies. In line with this, in a currently preferred embodiment of the disclosure, a sequencing library is composed of a plurality of PCR-amplified DNA molecules, fused with adapter molecules (or adapter sequences) compatible with a given NGS strategy of choice. A comprehensive overview of sequencing library types and ways of preparing them can be found in a review of van Dijk et al. Exp Cell Res. 2014.

Similarly, as used herein, the term *"means for preparing a nucleic acid library*" shall be understood as minimum necessary elements for preparing a NGS-suitable nucleic acid library, which include at least a compartment where a nucleic acid received from a source of nucleic acid can be subjected to nucleic acid library preparation; the minimum necessary reagents for the library preparation (such as appropriate enzyme or a mix of enzymes, NGS-strategy-specific adapter sequences or adapters, buffer etc.); and also a standard known in the art machinery and software suitable for of e.g. initiating and/or directing the library preparation procedure. As used herein the term *"reagents necessary for preparing a nucleic acid library*" is to be understood as any mix of reagents known in the art that are sufficient for preparing a nucleic library that can be used for NGS. Preferably, *"reagents necessary for preparing a nucleic acid library*" can to be construed as any mix of reagents known in the art that are sufficient for preparing a nucleic library that can be directly used for NGS, i.e. comprising NGS-specific adapter sequences that are compatible with a given NGS strategy of choice. Such reagents may comprise primer sequences wherein the NGS-specific adapter sequences are included in the sequences of primers and the reagent mix comprises enzymes, substrates, and buffering conditions which allow to perform a library PCR with such primers. Alternatively, such NGS-specific adapter sequences can be suitable for ligation and can be provided in a reagent mix also comprising enzyme ligase.

Further, the term *"means for liberating or purifying nucleic acid from the biological sample"* is to be understood as any plurality or chemical reagents and/or physical elements as known in the art that are known to be used for liberating nucleic acids from cells or other structures in a biological sample, and, in case of purification, sufficiently separating said nucleic acids from unwanted sample debris into an acceptably pure form (wherein the term "acceptably" depends on the further purpose of such purified nucleic acids), usually in an aqueous solution. Chemical reagents suitable for such purpose include e.g. any known in the art detergents and/or buffers comprising detergents, chaotropic agents, nuclease inhibitors etc. that are used in tissue or cell disrupting and/or liquefying, and thus releasing nucleic acids contained therein into solution. Similarly, physical elements known in the art to be used in various methods of sample processing for the purpose of nucleic acid liberation/purification include e.g. silica solid supports such as resins in spin columns, silica membranes, beads etc.; further mechanical disruptors or machines generating disruptive energy such as sonicators etc.

Then, the term *"nucleic acid*" and its equivalent *"polynucleotide",* as used herein, refer to a polymer of ribonucleosides or deoxyribonucleosides comprising phosphodiester linkages between nucleotide subunits. Nucleic acids include but are not limited to DNA and RNA, e.g. including genomic DNA, mitochondrial or meDNA, cDNA, mRNA, rRNA, tRNA, hnRNA, microRNA, IncRNA, and various modified versions thereof. Nucleic acids can most commonly be obtained from natural sources like biological samples obtained from different types of organisms. On the other hand, nucleic acids can also be synthesized, recombined, or otherwise produced in any of the known human-devised methods (e.g. nucleic acid amplification method like PCR).

As used herein, the term *"source of a nucleic acid*" is to be understood as any substance whether liquid or solid, comprising or expected to comprise nucleic acid. A source of nucleic acid can e.g. be an artificially created solution comprising a synthetic or recombinant nucleic acid such as among many other a solution containing a ligation product, an electrophoresis marker (so called "ladder"), a primer stock etc. Most commonly however, a source of nucleic acid will be a biological sample obtained from an organism or cells forming or derived thereof, preferably a clinical sample obtained from a patient.

As used herein, the term *"biological sample",* or simply *"sample",* is intended to include a variety of biological sources that contain nucleic acid and/or cellular material, for example including: cultures of cells such as mammalian cells but also of eukaryotic microorganisms, body fluids, body fluid precipitates, lavage specimen, fine needle aspirates, biopsy samples, tissue samples, cancer cells, other types of cells obtained from a patient, cells from a tissue or *in vitro* cultured cells from an individual being tested and/or treated for disease or infection, or forensic samples. Non-limiting examples of body fluid samples include whole blood, bone marrow, cerebrospinal fluid (CSF), peritoneal fluid, pleural fluid, lymph fluid, serum, plasma, urine, chyle, stool, ejaculate, sputum, nipple aspirate, saliva, swabs specimen, wash or lavage fluid and/or brush specimens.

Once a biological sample is provided into the systems or during performing the methods of the application, it will usually be contacted with a composition to provide a lysate in which nucleic acid is released. As used herein, by *"contacting"* is meant bringing together, exposing, incubating, or mixing of the sample and the composition. *"Releasing"* refers to liberating, obtaining and/or reversal of cross-linking. For liberating nucleic acid from a sample, protease activity and pH-buffering may be required from the composition. Releasing may require from the composition potential precipitating activity of components other than nucleic acid present in the investigated sample and removal/dissolving of fixative. Releasing may require conditions such as heating or High-Intensity Focused Ultrasound (HIFU). In one embodiment of the application, a biological sample is introduced into a cartridge compatible with an automated system such as a diagnostic analyzer, wherein the sample processing steps involving contacting with various solutions and releasing of nucleic acids take place.

Further, the term *"cartridge"* is to be understood as a self-contained assembly of chambers and/or channels, which is formed as a single object that can be transferred or moved as one fitting inside or outside of a larger instrument suitable for accepting or connecting to such cartridge. Some parts contained in the cartridge may be firmly connected whereas others may be flexibly connected and movable with respect to other components of the cartridge. Analogously, as used herein the term *"fluidic cartridge"* shall be understood as a cartridge including at least one chamber or channel suitable for treating, processing, discharging, or analyzing a fluid, preferably a liquid. In preferred embodiments of the present disclosure, a cartridge can additionally be equipped in means of physical or mechanical manipulation of received therein samples that aid their lysis and liquefaction. For example, an advantageous cartridge can comprise one-way inlet valve, a septum, one or more filters or affinity membranes, an overflow, and/or a zone compatible with HIFU treatment. Further, an advantageous cartridge can comprise means for mixing and/or proper distribution of contained therein liquids, such as one or more plungers or manifolds. Next, an advantageous cartridge may comprise several reaction and/or waste chambers, may provide for keeping the received sample fully contained from the external environment, or be made from affordable materials such as plastics and/or other polymers. An example of a suitable cartridge is given in WO2007004103. Advantageously, a fluidic cartridge can be a microfluidic cartridge. In the context of fluidic cartridges the terms *"downstream"* and *"upstream"* can be defined as relating to the direction in which fluids flow in such cartridge. Namely, a section of a fluidic path in a cartridge from which a fluid flows towards a second section in the same cartridge is to be interpreted as positioned *upstream* of the latter. Analogously, the section to which a fluid arrives later is positioned *downstream* with respect to a section which said fluid passed earlier.

In general, as used herein the terms *"fluidic"* or sometimes *"microfluidic"* refers to systems and arrangements dealing with the behavior, control, and manipulation of fluids that are geometrically constrained to a small, typically sub-millimeter-scale in at least one or two dimensions (e.g. width and height or a channel). Such small-volume fluids are moved, mixed, separated or otherwise processed at micro scale requiring small size and low energy consumption. Microfluidic systems include structures such as micro pneumatic systems (pressure sources, liquid pumps, micro valves, etc.) and microfluidic structures for the handling of micro, nano- and picoliter volumes (microfluidic channels, etc.). Exemplary microfluidic systems have been described in EP1896180, EP1904234, and EP2419705 and can accordingly be incorporated applied in certain embodiments of the presented herein application.

### BRIEF DESCRIPTION OF FIGURES

For a fuller understanding of the nature of the present disclosure, reference is made to the following detailed description taken in conjunction with the accompanying drawings in which:
**Figure 1****:** shows 5 DNA bands on an electrophoretic gel, corresponding to 5 products of a 5plex qPCR performed on a liquefied FFPE sample.
**Figure 2****:** shows qPCR amplification curves for the 5 products of the 5plex qPCR shown in Figure 1.
**Figure 3****:** shows a Cq to copy number histogram for at least 4 replicates of each of the 5plex qPCR products.
**Figure 4****:** shows R squared value determination for each of the 5plex qPCR products.
**Figure 5****:** shows the ability of the 5plex qPCR to distinguish between different degrees of nucleic acid fragmentation. Panel A shows results obtained from 3 FFPE samples having relatively intact DNA; Panel B shows results from another set of 3 FFPE samples with a higher level of fragmentation; lastly, Panel C shows results from 6 different FFPE samples containing heavily fragmented DNA.
**Figure 6****:** shows results of a BRAF-specific qPCR capable of discerning between wt and V600K/R/M BRAF mutants, performed on three FFPE samples each spiked with a plasmid containing a sequence encoding for either wt BRAF, V600M mutant BRAF, or V600K and T149C double-mutant BRAF.
**Figure 7****:** shows principles of a one type of NGS-ready library preparation using library PCR with primers containing NGS-specific adapters. Sequences of wild type (Seq ID NO.: 1), V600M (Seq ID NO.: 2) and V600K + V1794C (Seq ID NO.: 3) are represented.
**Figure 8****:** shows results of NGS performed on three FFPE samples each spiked with a plasmid containing a sequence encoding for either wt BRAF, V600M mutant BRAF, or V600K and T149C double-mutant BRAF.
**Figure 9****:** shows an example of an optimized sample-to-result workflow according to the present application.

### DETAILED DESCRIPTION OF THE INVENTION

The present invention generally relates to automatic preparation of next-generation sequencing (NGS) libraries from biological samples. In particular, the application relates to a generic way to generate a NGS-library from any type of sample (blood, stool, urine, FFPE, swab, ...) in an automated manner and with the possibility of automatically verifying quantity and quality of the thus obtained library or the nucleic acid used for its construction using a QC qPCR and of almost instantaneously obtaining clinical information from said nucleic acid using an assay qPCR. In case if the latter, it should be noted that exactly the same and identically-processed nucleic acid pool is used for NGS library preparation and qPCR, which further provides unique means for directly comparing the diagnostic information on key target mutations as screened by assay qPCR with a broader genetic landscape as later obtained from the NGS data.

In particular, the present invention provides a fluidic cartridge for automated processing a biological sample on an automated system and engageble with said system , the cartridge comprising:
- a sample compartment for receiving a biological sample;
- means for liberating or purifying nucleic acid from the biological sample received in the sample compartment, said means capable of entering in fluid communication with said sample compartment either by being connected by a channel to said sample compartment or by being entirely or partially positioned in the sample compartment;
- a library compartment for preparing a nucleic acid library positioned downstream of the sample compartment and of the means for liberating or purifying nucleic acid, the library compartment configured to receive at least a portion of the liberated or purified nucleic acid (e.g. as a result of a pumping action directing the extracted nucleic acids in a channel to the library compartment ) and comprising reagents for preparing a nucleic acid library such as enzymes, NGS-specific adapters, any buffering solutions etc.

One of the objects of the present invention is to provide means for biological sample-to-NGS library processing that is fast, easy-to-use, and requires minimal or virtually no human handling at least from the moment a sample is inserted into the cartridge of the present invention, the cartridge is engaged with its operating automated system, and the automated system is instructed to run the automated library preparation procedure. Thus, it is also one of the objectives of the present invention to provide a cartridge or a platform wherein the library preparation process does not require any human intervention from the moment a biological sample is provided (e.g. inserted into the cartridge or otherwise introduced into the automated system compatible with the cartridge) and the automated processing is initiated by the user.

Advantageously, the cartridge of the invention not only automatically prepares a NGS library but also is capable of concomitantly providing quantity and quality information related to said library, and/or also performing some clinical characterization of the sample from which the library is being generated. To achieve this, the present invention further provides a cartridge where in parallel with the library preparation, a real-time PCR (qPCR) can be thermocycled and monitored e.g. through a transparent wall. Thus, in a preferred embodiment a cartridge is provided that further comprises at least one thermocycling qPCR compartment positioned downstream of the sample compartment and of the means for liberating or purifying nucleic acid, and configured to receive at least a portion of the liberated or purified nucleic acid or at least a portion of the nucleic acid library prepared in the library compartment (e.g. by pumping through channels), wherein said thermocycling qPCR compartment is suitable for amplifying nucleic acids and allowing detection of signals generated during such amplification and comprises reagents for performing qPCR, such as at least one set of primers, a signal-generating dye or probe, a DNA polymerase, dNTPs, buffer, etc.

Preferably, the qPCR performed in the thermocycling qPCR compartment is a multiplex qPCR, i.e. a qPCR simultaneously amplifying and detecting multiple sequences in a single reaction. A multiplex qPCR uses multiple primer sets in a single qPCR mixture and thus generates multiple products in one tube, chamber, or other type of a qPCR thermocycling compartment. Multiplex qPCR using two primer sets (usually pairs) is called a duplex, often denoted 2plex. Similarly, a multiplex qPCR with three primer sets is a triplex or a 3plex. Preferred in the present disclosure multiplex qPCR arrangements include a 2plex, a 3plex, a 4plex, a 5plex, a 6plex, a 7plex, or more.

Therefore, preferably, the reagents for performing PCR comprise a plurality of primer sets, each directed to a different amplicon, wherein said plurality is preferably 2, 3, 4, 5, 6, 7, or more.

It is well known in the art that designing a robust multiplex qPCR is not easy as the assay requires that the multiplicity of individual primer sets (usually primer pairs) will specifically target their unique amplicons in one reaction tube and thus under a single set of reaction conditions. Primer design will typically take into account primer purity (17 to 30 bases in length); balance G/C and A/T-rich domains (20 to 70% G+C); set melting temperature between 55-80°C; avoid creating complementary 3'end base pairs; avoid primer self-complementary; and avoid 1 or more C's or G's at the 3' end of primers, especially when the multiplex is to be performed on complex samples such as eukaryotic genomic DNA. Several web-based primer design software tools are available that help design PCR primers (e.g. Primer3Plus). Other factors, such as the relative concentration of the primers, the right concentration of the PCR buffer components, balance between the magnesium chloride and deoxynucleotide concentrations, cycling temperatures, and DNA thermocycling polymerase etc. also often have to be fine-tuned for a successful multiplex qPCR. Notably, finding an optimal combination of the annealing temperature and buffer concentration is essential in multiplex PCR to obtain highly specific amplification products. Magnesium chloride concentration needs to be proportional to the amount of dNTP, while primer concentration for each target should be relatively robust. A choice of a proper polymerase can also have impact on the outcome of the reaction. In theory, multiplex PCR can be performed with standard PCR polymerase; however in practice it is preferred that highly processive and sensitive DNA polymerases such as GoTaq or AmpliTaq are used. For particularly sensitive applications, further modifications of multiplex qPCR such as the ones using DNAzymes or MNAzymes may be advantageous for the application in the present disclosure. The list of the mentioned-herein factors and multiplex optimization strategies is by no means to be interpreted as extensive, which will be immediately appreciated by any person skilled in the art.

In preferred embodiments of the disclosure, the quantification of the qPCR in the systems and methods of the application is based on the standard curve method; however, other quantification strategies can also be used, as will be immediately appreciated by any skilled person.

Similarly, many different general types of fluorescent dyes or detection probes can be used in the systems and methods of the present application. In preferred embodiments sequence specific probes will be used, e.g. selected from exonuclease probes, hybridization probes, or molecular beacons. Such probes not only add specificity to the assay, but are also key for enabling multiplex applications. As shown in the examples, the methods of the application were successfully applied with the use of Taqman probes but other probes would be suitable as well.

Advantageously, together with the NGS library preparation, the cartridge of the present disclosure provides for simultaneous qPCR screening of the provided therein clinical sample for the presence of therapy actionable alleles or disease-associated sequences. Some examples of such sequences, among many others, include mutant gene sequences associated with cancer, or exogenous pathogenic sequences indicative of an ongoing infection with a pathogenic organism. In line with this, in a preferred embodiment, a cartridge is provided, wherein the reagents for performing qPCR comprise at least one set of primers, preferably a plurality of sets of primers for amplifying at least one disease-associated target nucleic acid sequence.

In a further aspect of the present invention, the provided herein cartridge can also advantageously comprise means for assessing the quality of the purified nucleic acids or the already obtained library. Thus, in another preferred embodiment, a cartridge is provided wherein the reagents for performing qPCR comprise at least one set of primers, preferably a plurality of sets of primers for performing a quality control (QC) qPCR. In principle, for performing QC qPCR, targets can be selected from any genes or genomic regions. For diagnosis of genetically unstable conditions like cancer, it is better however, to avoid disease-sensitive regions that are likely to have their sequence mutated or change in copy number. Therefore, in a preferred embodiment, the targets of the quality control multiplex qPCR are selected from intra-exon sequences of single copy genes, such as a housekeeping gene. A further advantage of said solution is that the same target sequences directed to intra-exon regions can be used in assessment of both DNA as well as in RNA quality as a library material. Because of the latter, such design of the quality control multiplex qPCR multiplex of the present disclosure is particularly useful when it is desired to sequence both the genome and the transcriptome from one sample, which requires construction of and thus also quality verification for both DNA- and RNA-based. Thus, in one preferred embodiment, the quality control multiplex qPCR to be used in systems and method of the application, targets at least one intra-exon sequence in a single copy gene. Possibly, one, two, three, four, five, six, seven or more intra exon sequence in a one, two, three, four, five, six, seven or more single copy genes are targeted. Alternatively to housekeeping genes, in DNA library construction, repetitive sequences (e.g. LINEs, or SINEs such as Alu elements) may be the targets of interest. As a non-limiting example shown in the example section, the methods of the application are successfully practiced on intra-exon sequences of the RNaseP, HPRT1, Beta-Actin, TRFC and ABCB1 genes.

In one preferred embodiment, the cartridge of the invention can perform QC qPCR in addition to an assay qPCR that amplifies target disease-associated nucleic acid sequences. Thus, in an advantageous embodiment, a cartridge is provided further comprising at least a second one thermocycling qPCR compartment positioned downstream of the sample compartment and of the means for liberating or purifying nucleic acid, the at least second one thermocycling qPCR compartment configured to receive at least a portion of the liberated or purified nucleic acid or a portion of the nucleic acid library prepared in the library compartment, and comprising a at least one set of primers for performing a nucleic acid quality control (QC) qPCR.

It has been observed that a multiplex qPCR generating amplicons of different sizes is particularly suitable for assessing nucleic acid quality. Consequently, in another preferred embodiment, a cartridge is provided comprising a plurality of sets of primers for generating amplicons of different sizes. The size range of the amplicons varies from a smaller size Ax to a higher size Ay (with x<y), typically in the size range of 50-600 bp. Preferably the smaller Ax size ranges from 50 to 110 bp and can be of any length in between that range. Preferably Ax is between 60 bp +/- 10 bp. As shown further herein in Examples, Ax is 63 bp or 105 bp. Preferably the larger Ay size ranges from 300 to 550 bp and can be any length in between that range. As shown in further in the Examples, Ay is 504 bp (e.g. for the TRFC gene) or alternatively 318 bp (e.g. for the beta-actin gene). In case of RNA sequencing, particularly in applications focusing on microRNA (e.g. 15-35 bp size range), the smaller Ax size range may need to be lowered accordingly. As will be appreciated by any skilled artisan, during the multiplex qPCR design for the purposes of the present application, the preferred amplicon sizes may advantageously be selected in accordance with the NGS application of choice in order to provide the most adequate estimation about the NGS coverage according to amplicon length.

In preferred embodiments, the biological sample is a human clinical sample. The clinical sample can be a fresh sample, a fresh frozen sample, a fine needle aspirate, a sample that has been treated for preservation and may contain cross-linking of reactive sites due to fixation treatment, a wax-contacted or wax-embedded sample, an FFPE sample in the form of an FFPE slice, a liquid sample such as a urine sample, a blood sample, a serum sample, or any other clinical sample.

Once introduced to the cartridge of the invention, a biological sample will usually be processed by contacting it with a composition that provides for releasing of nucleic acids. In preferred embodiments, the composition is optimized for use in microfluidic analyzers and preferably contains surfactants rather than organic solvents. Mixing with said composition usually also facilitates transporting of such processed sample through a microfluidic system. In embodiments wherein the sample is an FFPE sample, the surfactant comprised in said composition will preferably be non-ionic. Nucleic acids obtained from FFPE samples typically contain nucleotide-to-nucleotide and nucleotide-to-protein cross-links, base modifications and other chemical modifications that affect the integrity of the nucleic acid. Preferred methods of the present disclosure incorporate a non-ionic surfactant and permit automated removal of embedded wax and liberation of the components without use of organic solvents. This is particularly beneficial because it puts the liberated nucleic acids in a condition and environment that interfaces with downscale applications requiring enzymatic activity such as nucleic acid amplification via PCR. In one embodiment, the lysate and/or components released from the sample will be further processed in diagnostic analyzers using microfluidic systems.

Optionally, the liberated nucleic acid is provided in a form sufficiently pure for being directly used as a template for a qPCR and a nucleic acid library construction according to the disclosure. In a preferred embodiment, the nucleic acid-liberating means performs its function in a fluidic or microfluidic arrangement. In such instance, the elements forming such nucleic acid-liberating means may comprise a series of consecutive or otherwise fluidly interconnected compartments, like chambers or channels, at least some of which being supplied with reagents like lysis buffers, enzyme solutions, extraction buffers, binding buffers and/or wash buffers; or optionally comprising any of the known in the art physical barriers, such as filters or high-affinity resins, that facilitate processes like mechanical sample clearing or nucleic acid binding, washing, and releasing. Such and alternative means for liberating nucleic acids are well known in the art and therefore will not be discussed herein in greater detail.

Usually, the fluidic cartridge of the invention will comprise means for dividing nucleic acids liberated and/or purified from a received therein sample between at least the library compartment and the thermocycling qPCR compartment. Such means could e.g. comprise two separate channels extending from the nucleic acid source-receiving compartment or the compartment whereto the liberated from said source nucleic acid is deposited in the last step of the nucleic acid liberation process, into the thermocycling qPCR compartment and into the library compartment, respectively. In order to actively transport the nucleic acid between the compartments of choice, the automated system of the disclosure could provide a pressure gradient capable of pushing or pulling a desired amount of fluid into prescribed direction. Generation of such pressure gradients by means of pumps, suction devices, manifolds etc. is widely employed in contemporary microfluidic systems and thus well known in the art.

As will be understood by any skilled person, fluidic or microfluidic cartridges suitable for the present application may contain at least two reaction chambers comprising the thermocycling qPCR compartment and the library compartment, and one or more fluid chambers. Some of the fluid chambers may hold fluid which is used for producing lysate from the sample. Other chambers may hold fluids such as washing fluids and amplification solution. Separate reaction chambers are used as the thermocycling qPCR compartment and the library compartment. The chamber configured to serve as the thermocycling qPCR compartment comprises a number of primer sets, along with other amplification reagents and enzymes required for performing a qPCR. The other chamber configured to serve as the library compartment is adapted to performing the steps of constructing a nucleic acid library for a NGS application of choice. Parts of the sample will be transferred to the reaction chambers and to make such transfer possible, chambers are connected to one or more fluid channel. In at least one, but preferably each of these fluid channels a valve means may be provided, which valve means preferably normally closes the fluid channel, but opens the fluid channel upon actuation of the valve means therewith placing the respective two chambers in fluid communication. The valve means may be designed as a one-way valve.

In another embodiment, the library compartment and the one or more of the thermocycling qPCR compartments of the present cartridge can be operated in the automated system engaging the cartridge simultaneously or sequentially. This means that three modes of operation can be envisaged: (i) both compartments operate once nucleic acid is fed into them, thus library preparation is independent of and proceeds in parallel with qPCR; and (ii) first, qPCR is made, then library is made; like this the decision to prepare a library for sequencing is made once the results of the qPCR are known and may depend on these results; (iii) first, the library is made, then qPCR is performed on the library for the verification of the library quality.

The option (i) above describes the simultaneous operation in which the preparation of a library for NGS from a part of a nucleic acid sample is run simultaneously with a qPCR assay on another part of the same nucleic acid sample, and both are preferably performed in parallel in a cartridge-based microfluidic system. As used herein the term *"simultaneously"* or *"in parallel*" refers to happening or being done at the same time. In such arrangement, the qPCR is being performed on a part from a nucleic acid sample at the same time as the nucleic acid library for NGS application is being constructed from another part from the same nucleic acid sample. In other words, during the simultaneous operation, both the sample analysis via qPCR and library construction are executed by the automated system of the invention at the same time.

Conversely, the options (ii) and (iii) above can both be described as operating "sequentially". In one possible embodiment of the sequential operation at least two thermocycling qPCR compartments operate on the automated system of the invention. For example, first qPCR can be done to read the expression of interesting markers and verify the quality of nucleic acid source fed into the system. Following this first qPCR (sequentially), or to save time in parallel with said first qPCR (simultaneously), a library is constructed. Then, a second or control qPCR can be performed on the thus constructed library to verify whether its quality is sufficient for subjecting it to further applications, such as sequencing.

With regard to the NG sequencing library preparation or construction, currently there exist many different ways of generating a sequencing-ready library, and their choice naturally depends on which NGS strategy is intended to be performed. In general, NGS library generation involves generation of nucleic acid fragments, which are compatible with given NGS. Therefore, in a preferred embodiment, a cartridge is provided wherein the reagents for preparing a nucleic acid library in the library allow:
- generation of nucleic acid fragments from the portion of the liberated or purified nucleic acid received into the library compartment, and
- attaching oligonucleotide adapters to at least one, preferably both ends of said generated nucleic acid fragments.

For most of the commercially available NGS platforms, amplification of nucleic acid fragments is necessary to generate sufficient copies of sequencing templates. Thus, preferably, a cartridge is provided wherein the generation of nucleic acid fragments is performed by a PCR, further referred to as "library PCR", and wherein the attaching oligonucleotide adapters to the library-PCR-generated nucleic acid fragments is performed by including an adapter sequence in a sequence of at least one primer used in said library PCR. Suitable library PCRs are known in the art and include methods such as bridge amplification or emulsion PCR.

As already state above, nucleic acid library construction is needed for DNA sequencing, RNA sequencing, and other applications such as sequencing-based methylation analysis. RNA sequencing (RNA-seq) is a method of investigating the transcriptome of an organism using deep-sequencing techniques. Total RNA generally contains only a very small percentage of coding or functional RNA; ribosomal RNA (rRNA: up to 80-90% of the total RNA), and to a lesser degree transfer RNA (tRNA), make up the majority of the RNA in a sample. Often, In order not to use 80-90% of one's sequencing capacity on repetitive rRNA sequences, rRNA can be removed from the sample prior to sequencing. The RNA after removal of rRNA is made into a library. This involves creating double-stranded cDNA through reverse transcription from the RNA (or fragmented RNA). This double-stranded cDNA may then be handled as normal genomic DNA throughout the remaining library construction process, including linking it with appropriate NGS-strategy specific adapters..

As the cartridges of present disclosure provide a convenient way for generating parallel datasets from several assays including NGS, sample quality assessment by QC qPCR, and possibly several target specific qPCR assays, in a particularly practical embodiment, cartridges of the present disclosure may advantageously be equipped with an element, i.e. an identifier, capable of storing cartridge- or patient-specific information, which would allow to easily keep track of all the assays performed on one cartridge or on samples from one patients. Such identifier could for example be a cartridge-unique code or a barcode containing data like a cartridge number, its batch or series number, production and expiry dates, types of target specific assays that the cartridge carries etc. Alternatively, the identifier could be not only readable but also writable storage medium like a disc or a microchip, where a patient-specific data (like name, age, date of the assay etc.) could be introduced, stored, and then retrieved at will in order to facilitate automated patient identification and minimize mixing of samples. The identifier could be conveniently scanned or otherwise introduced into the automated system engaging with and processing such cartridge, which would save the time of the operator performing the run on typing the required data and also eliminate a stage in the workflow where a human error can be introduced. Furthermore, thanks to the association of runs with one specific cartridge-identifier or patient-identifier, different tests performed on samples from the same patient could become easily traceable, retrievable, and associated with one another also in an automated way. Thus in an advantageous embodiment, the present application provides a cartridge in accordance with the previous embodiments, further comprising a cartridge-specific identifier like a unique code, barcode, or a microchip etc., for storing cartridge-specific information.

In another aspect, a cartridge is provided further comprising a recovery compartment positioned downstream of the sample compartment, said recovery compartment providing for recovering any of the following:
- a portion of the biological sample received into the sample compartment;
- a portion of the nucleic acid liberated or purified from the biological sample received into the sample compartment;
- at least a portion of the nucleic acid library prepared in the library compartment

Such recovery compartment may comprise or simply be made of another chamber wherein no reaction takes place during the operation of the system of the disclosure. Such recovery would preferably be easily accessible from outside the present automated system or a cartridge of the automated system. For example, it could comprise a wall made of a pierceable material (e.g. a foil or a film) that can be pierced by a needle of a syringe or a pipette, allowing aspiration of its contents. Alternatively, the recovery compartment could be selectively brought in fluid communication and filled in with any of the above by means on pumping and following instructions given by the user through an interface of the automated system of the disclosure.

In a possible embodiment, such recovery compartment could be an external container e.g. plastic tube or a vial, engageable with or connectable to the automated system of the disclosure. In such instance, any of the compartments as follows:
- a compartment housing at least a part of the biological sample introduced into the cartridge or at least a part of the nucleic acid liberated from said sample;
- the library compartment;
- at least one thermocycling qPCR compartment;
could comprise a structure (e.g. an extension like a channel or an zone engageable with an element forming a channel) capable of brining it in fluid communication with the recovery compartment by any means capable of transporting at least a part of the content comprised in any one of said above-listed compartments into the recovery compartments.

In an advantageous embodiment, the library compartment can comprise a structure capable of brining it in fluid communication directly with a compartment where NGS is performed, possibly wherein said compartment is comprised in another subsystem comprising an automatic sequencer being part of the automated system processing the cartridge.

In a further aspect, the present invention also provides an automated method for preparing a next-generation sequencing (NGS) nucleic acid library from a biological sample, said method comprising the steps of:
a) receiving a biological sample;
b) liberating or purifying at least a part of nucleic acids from the received biological sample;
c) providing at least a portion of the liberated or purified nucleic acid into a library compartment for preparing a NGS nucleic acid library and comprising reagents for preparing a nucleic acid library; and
d) preparing a NGS nucleic acid library;
wherein at least the steps b) to d) are performed on an automated system without any human manual intervention, at least after the biological sample has been received in said automated system.

In a preferred embodiment, the step of preparing a NGS nucleic acid library is performed by PCR.

In another particularly preferred embodiment in accordance with the above embodiments of the method of the application, the method further comprises the step of performing qPCR on said automated system either sequentially or simultaneously with respect to the step of preparing the NGS nucleic acid library, wherein said qPCR is performed on a second portion of the liberated or purified nucleic in a thermocycling qPCR compartment suitable for amplifying nucleic acids and allowing detection of signals generated during such amplification.

As explained above, one of the aspects of the invention involves preparing a NGS library concomitantly with running either an assay qPCR and/or a QC qPCR using nucleic acid obtained from the same sample. Consequently, another embodiment, a method is provided wherein said at least one qPCR amplifies at least one disease-associated target nucleic acid sequence, or is a quality control (QC) qPCR.

In a particularly preferred embodiment, the method of the invention is performed on a cartridge engageable with the automated system. Thus, most preferably the afore mentioned steps of obtaining nucleic acid from a sample, NGS library preparation, assay qPCR, and QC PCR are performed in one cartridge, preferably being a fluidic or microfluidic cartridge engageable with an analyzer-type apparatus so that the cartridge is a self-contained disposable platform for performing the steps of the method according to the present application. In such advantageous embodiment, all of the required reagents are pre-positioned within such cartridge, for storage considerations preferably in a dried-down or a lyophilized form.

In another preferred embodiment, for the advantageous considerations as described above, desirably a method is provided wherein the cartridge comprises a cartridge-specific identifier for storing cartridge-specific information and wherein the method further comprises the step of introducing to the automated system the information stored in the cartridge-specific identifier.

In summary, the present invention provides an effective automation of workflow with integrated "sample-in, quality checked nucleic acid library-out" strategy. The presented herein approach has a great potential for providing a minimal turn-around times, lower costs, and improved NGS success rates. Furthermore, the cartridges, automated systems, and methods of the disclosure not only afford for simplification and acceleration of the library construction workflow, but they are also universally adaptable for the use with different sample types, even the challenging ones like FFPE samples. Thus, in a final aspect of the invention, use is provided of cartridges, automated systems, and methods of the invention for automated preparation of a next-generation sequencing (NGS) library from a biological sample.

It is to be understood that both the foregoing general description and detailed description are only exemplary and explanatory and are not restrictive for the invention as claimed. In this application, the use of singular includes the plural unless specifically stated otherwise. In this application the use of "or" means "and/or" unless stated otherwise. The use of the terms "including", "includes" or "included" is not limiting.

### EXAMPLES

### Example 1: Development of a QC qPCR for an automated sample-to-output assessment of nucleic acids

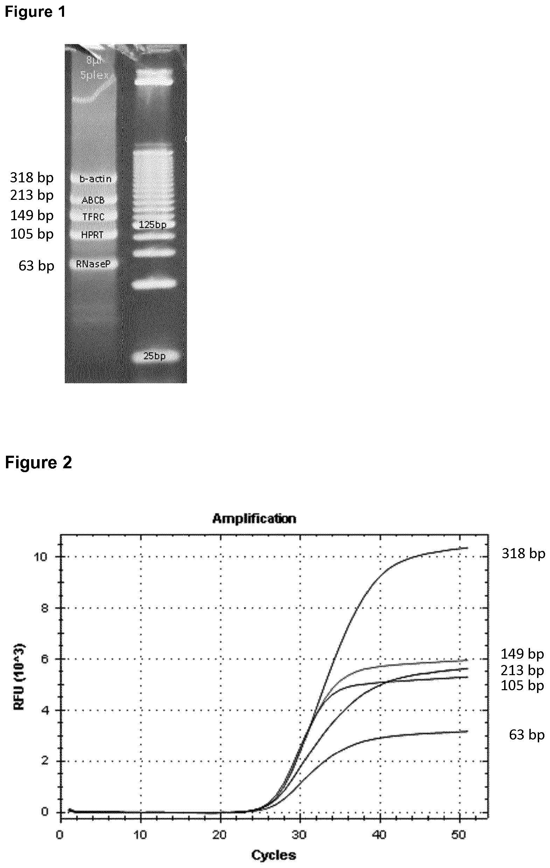

First, a quality control (QC) qPCR assay was developed for the purpose of assessing the amount and quality of nucleic acids present in a sample in a fully automated manner. The present QC qPCR tests for the presence of amplicons of various lengths, each derived from a different single copy human gene, and serves to assess nucleic acid suitability for NGS application. The amplicons and their lengths are as follows: (1) 63bp fragment from human RNaseP gene; (2) 105bp fragment from HPRT; (3) 149bp fragment from TFRC; (4) 213bp fragment from ABCB; and (5) 318bp fragment from β-actin. The amplification of the fragments in one PCR reaction (5plex) was initially verified using a qPCR performed on a liquefied FFPE sample (Horizon FFPE sample) with a GoTaq polymerase and Taqman probes (composition as specified by the supplier). The qPCR programme was 5' hold 95°C, followed by 50 cycles of 5" 95°C 44" 64°C. Figure 1 shows the obtained fragments (left lane) next to a DNA ladder (right lane) on a SYBR green stained 10% polyacrylamide gel following electrophoresis in TBE. The corresponding qPCR profile of the same sample shown in Figure 2 (sizes of amplicons indicated next to the corresponding curves). The Cq values determined with the regression algorithm contained within the Biorad CFX Manager 3.1 are : (1) 26.1 for the 63bp fragment (RNaseP); (2) 25.6 for the 105bp fragment (HPRT); (3) 26.0 for the 149bp fragment (TFRC); (4) 26.2 for the 213bp fragment (ABCB); and (5) 27.4 for the 318bp fragment (β-actin).

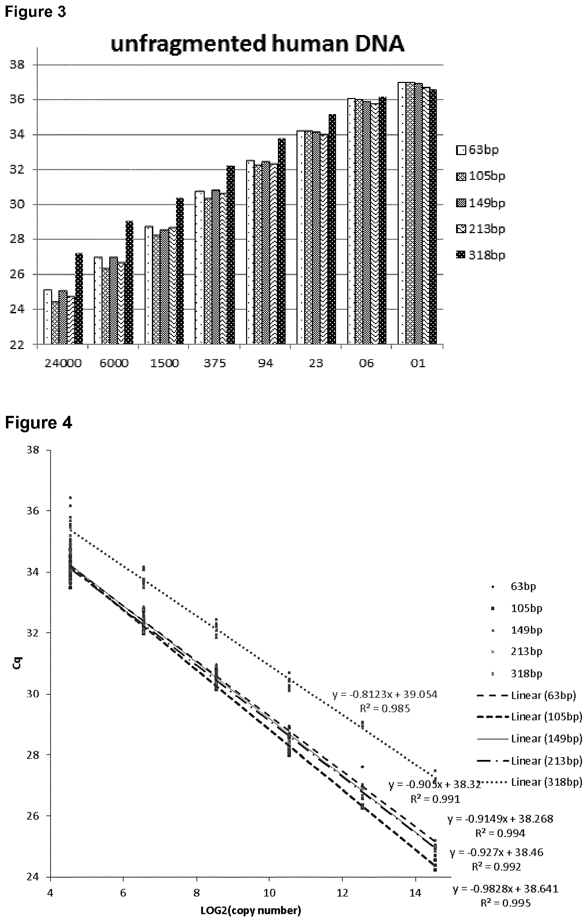

Next, the 5plex performance on unfragmented human genomic DNA was assessed to obtain standard curves with R squared values for each of the 5 amplicons. To do so, non-fragmented human genomic DNA at 173µg/ml (Promega) was used as a substrate and the copy number was deduced using 3.3pg/haploid genome as a premise. 4 replicates at 24000 copies per PCR, 4 replicates at 6000 copies, 8 replicates at 1500 copies, 8 replicates at 375 copies, 12 replicates at 94, 16 replicates at 23 copies, 20 replicates at 5.9 copies and 24 replicates at 1.5 copies per PCR were amplified and the Cqs determined as described above. The median Cq values were determined while omitting non-amplifications (so called flatliners). The histogram representing this experiment is shown in Figure 3. The standard curves were deduced for each amplicon using logarithmic regression and the R squared value was determined as exemplified in Figure 4 for the complete dataset and the dataset without the Cq values from both 5.9 and 1.5 copies per PCR, respectively. As expected, the R squared values approach 1 better with only data points in double digits copy number. For Cq values below 34, equations with the highest R squared values were used. Notably, for Cq values above 34, the quantification is known to be less accurate due to stochastic effects. The thus calculated according to said equations copy numbers of each of the 5 amplicons allow for the determination of both the useful DNA content and the degree of nucleic acid fragmentation in a given sample. An analysis of the direction coefficient of the linear regression between the log2(copy number input) and the Cq provides further indication of the efficiency of amplification in 5plex qPCR of each of the amplicons. As known in the art, a perfect qPCR would be assumed to double amount of amplicon (and hence also the net Taqman fluorescence) per cycle, leaving an absolute direction coefficient of 1. In line with this, as shown in Figure 4, the absolute direction coefficients for all amplicons except for the largest amplicon of 318bp are 0.9 or higher, indicating a robust amplification close to doubling Taqman probe degradation per PCR cycle. The largest amplicons size shows >0.8 absolute direction coefficient indicating that the largest fragment amplifies slower and that there is little point in designing a PCR QC test with longer amplicons for this type of Taqman probe-based assay.

### Example 2. Automated FFPE sample processing, nucleic acid quality assessment, target actionable marker screening, and library construction

For the purpose of demonstrating the feasibility of the present disclosure, a set of Biocartis Idylla cartridges was prepared, each cartridge comprising in separate PCR chambers: (i) reagents for performing the above-described QC 5plex qPCR, (ii) reagents for performing target qPCR for detecting wt and V600M/R mutant BRAF, and (iii) reagents for constructing a DNA library compatible with Illumina MiSeq sequencer. Next, a set of FFPE samples to be analyzed on said cartridges were spiked with plasmids encoding for human BRAF. To simulate clinical reality, different BRAF sequences were used including a fragment containing a wild type (wt) BRAF sequence, a fragment encoding for a V600M mutation, and a fragment encoding for two mutations V600K and T149C. The two mutated fragments were spiked in different amounts with respect to the amount of the wild type copies present in the FFPE samples to obtain a relative concentration of 10% and 5%, respectively. Each of the different BRAF-spiked FFPE samples was introduced into a separate cartridge and processed in a fully automated manner on the Biocartis Idylla instrument. In brief, the processing involved sample liquefaction (as described in e.g. WO2014128129), followed by nucleic acid purification on a silica membrane provided in the cartridge, and then followed by three independent and individually-controlled PCR reactions performed in parallel, which included: (i) verification of the quality of the purified nucleic acids via quality control 5plex QC q PCR, as described above; (ii) real time detection of selected BRAF targets (qPCR for target actionable mutations); and (iii) construction of a DNA library using BRAF-specific or standard random-priming primers comprising linkers compatible with Illumina MiSeq sequencer. The latter library-construction PCR was performed using a Q5 high fidelity hot start polymerase (New England Biolabs) and cycled according to the following program: 5' at 95°C and 50 cycles of 90" at 60°C, 5" at 94°C.

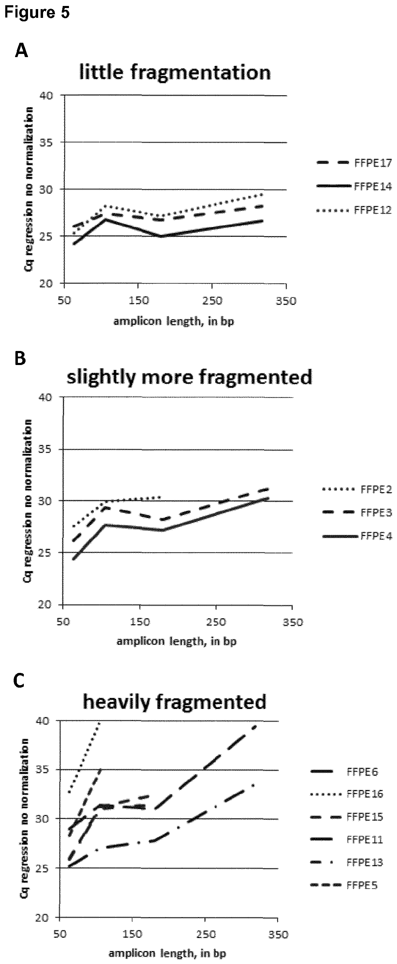

Figure 5 shows the results of the 5plex QC qPCR on the different FFPE samples, which provide information with regard to the integrity of the DNA present in said samples. Panel A shows three examples of FFPE tissue samples that contain relatively intact DNA. Panel B shows three other examples that have a slightly higher degree of DNA fragmentation. Lastly, Panel C shows 6 examples of FFPE tissue samples that contain heavily fragmented DNA, which is a counter-indication for subjecting such samples to further analysis by NGS.

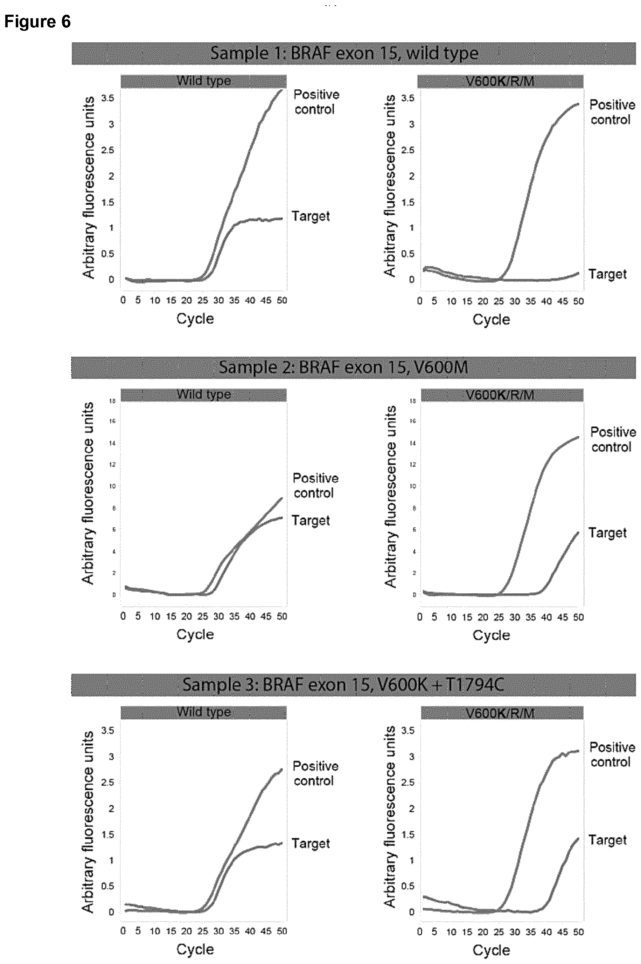

Based on the results of the 5plex QC qPCR, three samples with relatively intact DNA and containing three different forms of spiked BRAF (wt, V600M mutant, or double mutant V600K + T149C) were selected further investigation. Firstly, the results obtained from the assay qPCR capable of detecting wt BRAF and V600M BRAF mutation were checked to confirm the presence of the correct BRAF form. The results are shown in Figure 6 They demonstrate that in all of the screened three FFPE samples, wt BRAF signal could be detected (Figure 6, left column, the term "target" refers to wt BRAF sequence). This result was expected as all FFPE samples prior to spiking with different BRAF plasmids were known to contain wt genomic BRAF sequence. Concerning the detection of the V600M mutant (Figure 6, right column, the term "target" refers to V600M/K BRAF sequence), as expected, in the sample spiked only with the wt BRAF-encoding plasmid, no V600M mutant could be detected (Figure 6, top right pane; flat signal line for the target). However, in the FFPE samples spiked with either the V600M mutant, or the double mutant V600K + T149C, the mutation V600M was correctly detected at the expected amounts (Figure 6, right column, bottom and middle pane). Because the used-herein BRAF-specific qPCR did not include a specific probe for the T1794C mutation, said mutation could not be detected in the double mutant BRAF-spiked sample.

### Example 3. Sequencing of the selected NGS libraries

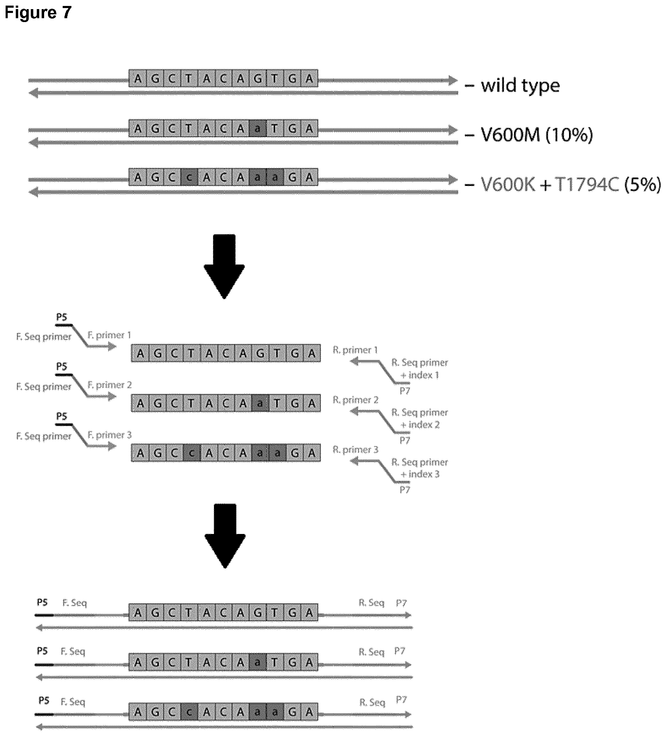

To confirm the results of the BRAF-specific qPCR and to also detect the presence of the undetected T1794C mutation, the NGS libraries constructed from the same three selected FFPE samples plasmid (wt, V600M, or V600K and T149C) were then subjected to Illumina MiSeq sequencing. The library PCR used for constructing these libraries is schematically shown in Figure 7 and, as mentioned above, was performed on the same cartridges as and in parallel with the 5ples QC PCR and the BRAF-specific assay qPCR. The library PCR included 50 cycles and used simplified BRAF-specific fusion primers (also known as tailed primers). The fusion primers (shown in middle pane of Figure 7) in addition to the target (BRAF)-specific sequence also contained sequencing primer sequence and a tag (P5 and P7) for flow cell attachment. In addition, the reverse primer also contained a barcode (or and index) that during a sequencing run allows to discern between samples obtained from different sources. The reason for introducing such barcode is that typically, libraries constructed from different samples or patients are pooled and sequenced on the same NGS instrument. Thus, in order to differentiate between libraries obtained from the three different FFPE samples, each cartridge contained a slightly different reverse primer having a unique barcode sequence.

Before sequencing, the three NGS libraries were recovered from respective cartridges using a needled syringe, after which the samples were pooled and purified further on the bench to remove any unreacted primers and primer-dimers. It should be noted that the latter purification step can also be performed automatically. Finally, the purified NGS-libraries were loaded into a flow cell of the MiSeq Illimina instrument and sequenced.

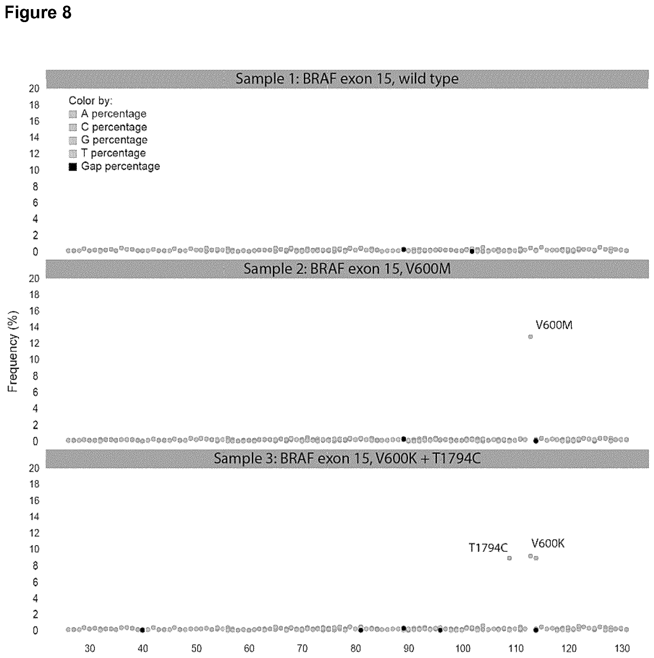

The results of the sequencing run are shown in Figure 8. In line with the afore-described results of the BRAF-specific qPCR, in the first sample that contained only wt BRAF no mutations were detected. For the two other samples, all the expected BRAF gene mutations were correctly identified during sequencing, even if they could not be captured in the BRAF-specific qPCR. In particular, NGS not only detected the T1794C BRAF mutation missed on the target-specific qPCR, but also allowed to discriminate between the V600M and V600K mutations in mutant- and double mutant-spiked FFPE samples, respectively, thus providing even more exact identification of the already detected mutations. The present results demonstrate the unprecedented robustness of the present disclosure, wherein desired results are not only provided in a fast and efficient way, but also can be successfully followed up at will if deeper insight is desired.

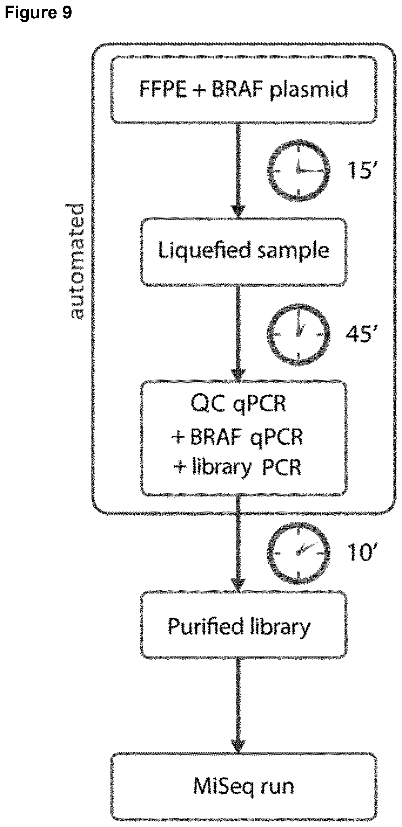

For the fuller appreciation of the present disclosure, the above-described workflow is schematically illustrated in Figure 9. It starts from providing an FFPE sample into a cartridge, after which the subsequent steps till obtaining of the final qPCR results (of both QC qPCR and target actionable marker qPCR, here BRAF) and ready-to-use library are performed in a fully automated and rapid manner (real time frames provided). It should be noted that all these results and the library are obtained from the same and identically-processed sample, which ensures high-comparability of the data from different assays. Notably, by concomitant NGS library construction and providing information with regard to said library's quality, the present approach not only allows to quickly subject a given sample to a NGS clinical follow-up, but also allows to decide whether such rather costly follow-up is feasible in view of that sample's quality. In view of the above, the present disclosure opens new possibilities in the current diagnostic practice.

### REFERENCES

Schiefer Anna-Iris et al. "Evaluation of a novel fully automated PCR-based technique for detection of BRAF-status in FFPE tissue", 1 January 2014.
"Beckman Coulter SPRIworks Systems", 1 January 2011.
"The SPRIworks Fragment Library System Simplifying Next Generation Sequencing Workflows SPRIworks Fragment Library System II Fully Automated Fragment Library System for the Roche GS FLX* DNA Sequencer", 1 January 2010.
Buehler B, Hogrefe HH, Scott G, Ravi H, Pabón-Peña C, O'Brien S, Formosa R, Happe S. Rapid quantification of DNA libraries for next-generation sequencing. Methods. 2010 Apr;50(4):S15-8.
Devonshire AS, Sanders R, Wilkes TM, Taylor MS, Foy CA, Huggett JF, Application of next generation qPCR and sequencing platforms to mRNA biomarker analysis, Methods 2013, 59(1): 89-100.

## Claims

1. A cartridge for automated processing of a biological sample, the cartridge comprising
- a sample compartment for receiving a biological sample comprising cellular material;
- means for liberating or purifying nucleic acid from the cellular material received in the sample compartment, said means comprising at least chemical reagents and being arranged to enter in fluid communication with said sample compartment;
the cartridge further comprising three separate compartments positioned downstream of the sample compartment and of the means for liberating or purifying nucleic acid, each of said three separate compartments being configured to receive a portion of the liberated or purified nucleic acid, wherein:
∘ the first of the three separate compartments is a first thermocycling qPCR compartment suitable for amplifying nucleic acids and for allowing detection of signals generated during such amplification and is comprising at least one set of primers for amplifying at least one disease-associated target nucleic acid sequence;
∘ the second of the three separate compartments is a second thermocycling qPCR compartment and is comprising a plurality of sets of primers for generating amplicons of different sizes that are adapted for performing a nucleic acid quality control (QC) qPCR;
∘ the third of the three separate compartments is a library compartment for preparing a next-generation sequencing (NGS) nucleic acid library defined as a set of polynucleotides ready for sequence analysis by an NGS strategy, the library compartment comprising reagents necessary for preparing the NGS nucleic acid library, said reagents comprising at least:
▪ means for generation of nucleic acid fragments from the portion of the liberated or purified nucleic acid received into the library compartment;
▪ oligonucleotide adapters suitable for the NGS strategy; and
▪ means for attaching the oligonucleotide adapters to at least one, preferably both ends of said generated nucleic acid fragments.

2. Cartridge according to claim 1, wherein the generation of nucleic acid fragments is performed by a PCR, further referred to as "library PCR", and
wherein the attaching of the oligonucleotide adapters to the library-PCR-generated nucleic acid fragments is performed by including an adapter sequence in a sequence of at least one primer used in said library PCR.

3. Cartridge according to any of the preceding claims, further comprising a cartridge-specific identifier for storing cartridge-specific information.

4. Cartridge according to any of the preceding claims, further comprising a recovery compartment positioned downstream of the sample compartment, said recovery compartment providing for recovering any of the following:
- a portion of the biological sample received into the sample compartment;
- a portion of the nucleic acid liberated or purified from the biological sample received into the sample compartment;
- at least a portion of the nucleic acid library prepared in the library compartment.

5. An automated method for preparing a next-generation sequencing (NGS) nucleic acid library from a biological sample, said method comprising the steps of:
a) receiving a biological sample comprising a cellular material into the cartridge according to any of claims 1-4;
b) liberating or purifying inside of said cartridge at least a part of nucleic acids from the received cellular material ;
c) providing a portion of the liberated or purified nucleic acid into three separate compartments of said cartridge and performing in each of the three separate compartments one of the three following reactions:
∘ a qPCR amplifying at least one disease-associated target nucleic acid sequence;
∘ a quality control (QC) qPCR generating amplicons of different sizes; and
∘ preparing of a next-generation sequencing (NGS) nucleic acid library defined as a set of polynucleotides ready for sequence analysis by an NGS strategy; the preparing comprising generating nucleic acid fragments from a portion of the liberated or purified nucleic acid, and attaching oligonucleotide adapters suitable for the NGS strategy to at least one, preferably both ends of said generated nucleic acid fragments,
wherein both of the qPCR amplifying at least one disease-associated target nucleic acid sequence and the QC qPCR are performed in compartments allowing detection of signals generated during qPCR amplification, and wherein both of said qPCRs are performed either sequentially or simultaneously with respect to the preparing of the NGS nucleic acid library.

6. Automated method according to claim 5, wherein the cartridge comprises a cartridge-specific identifier for storing cartridge-specific information and wherein the method further comprises the step of introducing to the automated system the information stored in the cartridge-specific identifier.

7. Use of a cartridge according to claims 1-5 for automated preparation of a next-generation sequencing (NGS) library from a biological sample.

## Patentansprüche

1. Eine Kartusche zur automatisierten Verarbeitung einer biologischen Probe; die Kartusche umfasst
- eine Probenkammer zum Aufnehmen einer biologischen Probe, die Zellmaterial umfasst;
- Mittel zum Freisetzen oder Reinigen von Nukleinsäure aus dem in der Probenkammer aufgenommenen Zellmaterial, wobei das Mittel mindestens chemische Reagenzien umfasst und so angeordnet ist, dass es mit der Probenkammer in Fluidverbindung tritt;
die Kartusche umfasst ferner drei separate Kammern, die der Probenkammer und der Mittel zum Freisetzen oder Reinigen von Nukleinsäure nachgelagert positioniert sind, wobei jede der drei separaten Kammern so konfiguriert ist, dass sie einen Teil der freigesetzten oder gereinigten Nukleinsäure aufnimmt, wobei:
∘ die erste der drei separaten Kammern eine erste Thermocycling-qPCR-Kammer ist, die zum Amplifizieren von Nukleinsäuren und zum Ermöglichen des Nachweises von während einer solchen Amplifikation erzeugten Signalen geeignet ist und mindestens einen Satz von Primern zum Amplifizieren mindestens einer krankheitsassoziierten Zielnukleinsäuresequenz umfasst;
∘ die zweite der drei separaten Kammern ist eine zweite Thermocycling-qPCR-Kammer und umfasst eine Vielzahl von Primersätzen zum Erzeugen von Amplikonen unterschiedlicher Größe, die zum Durchführen einer Nukleinsäure-Qualitätskontroll-(QC)-qPCR angepasst sind;
∘ die dritte der drei separaten Kammern ist eine Bibliothekskammer zur Vorbereitung einer Next-Generation-Sequenzierung (NGS) Nukleinsäurebibliothek, definiert als ein Satz von Polynukleotiden, die zur Sequenzanalyse durch eine NGS-Strategie bereit sind, wobei die Bibliothekskammer Reagenzien umfasst, die zum Vorbereiten der NGS-Nukleinsäurebibliothek erforderlich sind. Dabei umfassen die Reagenzien mindestens:
▪ Mittel zur Erzeugung von Nukleinsäurefragmenten aus dem Teil der freigesetzten oder gereinigten Nukleinsäure, die in die Bibliothekskammer aufgenommen wurde;
▪ für die NGS-Strategie geeignete Oligonukleotidadapter; und
▪ Mittel zum Befestigen der Oligonukleotidadapter an mindestens einem, vorzugsweise beiden Enden der erzeugten Nukleinsäurefragmente.

2. Kartusche nach Anspruch 1, wobei die Generierung von Nukleinsäurefragmenten durch eine PCR durchgeführt wird, die weiter als "Bibliotheks-PCR" bezeichnet wird, und
wobei das Anbringen der Oligonukleotid-Adapter an die Bibliothek-PCR-erzeugten Nukleinsäurefragmente durchgeführt wird, indem eine Adaptersequenz in eine Sequenz von mindestens einem Primer eingeschlossen wird, der in der Bibliothek-PCR verwendet wird.

3. Kartusche nach einem der vorangehenden Ansprüche, ferner umfassend eine kartuschenspezifische Kennung zum Speichern von kartuschenspezifischen Informationen.

4. Kartusche nach einem der vorangehenden Ansprüche, ferner umfassend eine Rückgewinnungskammer, die der Probenkammer nachgelagert positioniert ist, wobei die Rückgewinnungskammer die Rückgewinnung von einem der Folgenden bereitstellt:
- einen Teil der in die Probenkammer aufgenommenen biologischen Probe;
- einen Teil der aus der biologischen Probe freigesetzten oder gereinigten Nukleinsäure, die in die Probenkammer aufgenommen wurde;
- mindestens einen Teil der in der Bibliothekskammer hergestellten Nukleinsäurebibliothek.

5. Automatisiertes Verfahren zum Herstellen einer NGS-Nukleinsäurebibliothek (NGS = Next-Generation-Sequenzierung) aus einer biologischen Probe, wobei das Verfahren die folgenden Schritte umfasst:
a) Aufnehmen einer biologischen Probe, die ein Zellmaterial umfasst, in die Kartusche nach einem der Ansprüche 1-4;
b) Freisetzen oder Reinigen innerhalb der Kartusche mindestens eines Teils der Nukleinsäuren aus dem aufgenommenen Zellmaterial;
c) Bereitstellen eines Teils der freigesetzten oder gereinigten Nukleinsäure in drei separaten Kammern der Kartusche und Durchführen einer der drei folgenden Reaktionen in jeder der drei separaten Kammern:
∘ eine qPCR, die mindestens eine krankheitsassoziierte Zielnukleinsäuresequenz amplifiziert;
∘ eine Qualitätskontroll-(QC)-qPCR, die Amplikone unterschiedlicher Größe erzeugt; und
∘ Vorbereitung einer Next-Generation-Sequenzierung (NGS) Nukleinsäurebibliothek , definiert als ein Satz von Polynukleotiden, die für eine Sequenzanalyse durch eine NGS-Strategie bereit sind; wobei die Vorbereitung das Erzeugen von Nukleinsäurefragmenten aus einem Teil der freigesetzten oder gereinigten Nukleinsäure und das Anbringen von Oligonukleotidadaptern, die für die NGS-Strategie geeignet sind, an mindestens einem, vorzugsweise beiden Enden der erzeugten Nukleinsäurefragmente umfasst,
wobei sowohl die qPCR, die mindestens eine krankheitsassoziierte Zielnukleinsäuresequenz amplifiziert, als auch die QC-qPCR in Kammern durchgeführt werden, die den Nachweis von Signalen ermöglichen, die während der qPCR-Amplifikation erzeugt werden, und wobei beide der qPCRs entweder sequentiell oder gleichzeitig in Bezug auf die Vorbereitung der NGS-Nukleinsäurebibliothek durchgeführt werden.

6. Automatisiertes Verfahren nach Anspruch 5, wobei die Kartusche eine kartuschenspezifische Kennung zum Speichern kartuschenspezifischer Informationen umfasst und wobei das Verfahren ferner den Schritt des Einführens der in der kartuschenspezifischen Kennung gespeicherten Informationen in das automatisierte System umfasst.

7. Verwendung einer Kartusche nach den Ansprüchen 1-5 zur automatisierten Herstellung einer Next-Generation-Sequenzierung (NGS) bibliothek aus einer biologischen Probe.

## Revendications

1. Cartouche pour le traitement automatisé d'un échantillon biologique, la cartouche comprenant
- un compartiment à échantillons pour la réception d'un échantillon biologique comprenant du matériel cellulaire ;
- moyens de libération ou de purification d'acide nucléique à partir du matériau cellulaire reçu dans le compartiment à échantillons, ledit moyen comprenant au moins des réactifs chimiques et étant agencé pour entrer en communication fluidique avec ledit compartiment à échantillons ;
la cartouche comprenant en outre trois compartiments séparés positionnés en aval du compartiment à échantillons et du moyen de libération ou de purification d'acide nucléique, chacun desdits trois compartiments séparés étant configuré pour recevoir une portion de l'acide nucléique libéré ou purifié, dans laquelle :
∘ le premier des trois compartiments séparés est un premier compartiment thermocyclable qPCR adapté pour amplifier des acides nucléiques et pour permettre la détection de signaux générés durant une telle amplification et comprend au moins un ensemble d'amorces pour amplifier au moins une séquence d'acides nucléiques cibles associés à la maladie ;
∘ le deuxième des trois compartiments séparés est un deuxième compartiment thermocyclable qPCR et comprend une pluralité d'ensembles d'amorces pour générer des amplicons de différentes tailles qui sont adaptés pour effectuer un contrôle qualité d'acide nucléique (QC) qPCR ;
∘ le troisième des trois compartiments séparés est un compartiment de bibliothèque pour préparer une bibliothèque d'acides nucléiques de séquençage de nouvelle génération (NGS) définie comme un ensemble de polynucléotides prêts pour l'analyse de séquence par une stratégie NGS, le compartiment de bibliothèque comprenant des réactifs nécessaires à la préparation de la bibliothèque d'acides nucléiques NGS, lesdits réactifs comprenant au moins :
▪ moyens de génération de fragments d'acide nucléique provenant de la portion de l'acide nucléique libéré ou purifié reçue dans le compartiment de bibliothèque ;
▪ des adaptateurs d'oligonucléotide adaptés à la stratégie NGS ; et
▪ moyens de fixer les adaptateurs d'oligonucléotide à au moins une, de préférence aux deux extrémités desdits fragments d'acide nucléique générés.

2. Cartouche selon la revendication 1, dans laquelle la génération de fragments d'acide nucléique est effectuée par une PCR, également appelée « PCR de bibliothèque », et
dans laquelle la fixation des adaptateurs d'oligonucléotide aux fragments d'acide nucléique générés par PCR de bibliothèque est effectuée en incluant une séquence d'adaptateurs dans une séquence d'au moins une amorce utilisée dans ladite PCR de bibliothèque.

3. Cartouche selon l'une quelconque des revendications précédentes, comprenant en outre un identifiant spécifique à la cartouche pour stocker des informations spécifiques à la cartouche.

4. Cartouche selon l'une quelconque des revendications précédentes, comprenant en outre un compartiment de récupération placé en aval du compartiment à échantillons, ledit compartiment de récupération prévoyant la récupération de l'un quelconque des éléments suivants :
- une portion de l'échantillon biologique reçu dans le compartiment à échantillons ;
- une portion de l'acide nucléique libéré ou purifié de l'échantillon biologique reçu dans le compartiment à échantillons ;
- au moins une portion de la bibliothèque d'acides nucléiques préparée dans le compartiment de bibliothèque.

5. Procédé automatisé de préparation d'une bibliothèque d'acides nucléiques de séquençage de nouvelle génération (NGS) à partir d'un échantillon biologique, ledit procédé comprenant les étapes consistant à :
a) recevoir un échantillon biologique comprenant un matériau cellulaire dans la cartouche selon l'une quelconque des revendications 1-4 ;
b) libérer ou purifier, à l'intérieur de ladite cartouche, au moins une partie d'acides nucléiques du matériau cellulaire reçu ;
c) fournir une portion de l'acide nucléique libéré ou purifié dans trois compartiments séparés de ladite cartouche et effectuer dans chacun des trois compartiments séparés l'une des trois réactions suivantes :
∘ une qPCR amplifiant au moins une séquence d'acides nucléiques cibles associés à la maladie ;
∘ un contrôle qualité (QC) qPCR générant des amplicons de différentes tailles ; et
∘ la préparation d'une bibliothèque d'acides nucléiques de séquençage de nouvelle génération (NGS) définie comme un ensemble de polynucléotides prêts pour l'analyse de séquence par une stratégie NGS ; la préparation comprenant la génération de fragments d'acide nucléique à partir d'une portion de l'acide nucléique libéré ou purifié, et la fixation d'adaptateurs d'oligonucléotide adaptés à la stratégie NGS à au moins une, de préférence aux deux extrémités desdits fragments d'acide nucléique générés,
dans lequel tant la qPCR amplifiant au moins une séquence d'acides nucléiques cibles associés à la maladie que le QC qPCR sont effectués dans des compartiments permettant la détection de signaux générés pendant l'amplification qPCR, et dans lequel les deux desdites qPCR sont effectuées soit séquentiellement soit simultanément par rapport à la préparation de la bibliothèque d'acides nucléiques NGS.

6. Procédé automatisé selon la revendication 5, dans lequel la cartouche comprend un identifiant spécifique à la cartouche pour stocker des informations spécifiques à la cartouche et dans lequel le procédé comprend en outre l'étape d'introduction au système automatisé des informations stockées dans l'identifiant spécifique à la cartouche.

7. Utilisation d'une cartouche selon les revendications 1-5 pour la préparation automatisée d'une bibliothèque de séquençage de nouvelle génération (NGS) à partir d'un échantillon biologique.
